# EUROPEAN PATENT SPECIFICATION

(11) **EP 1 796 787 B1**
(45) Date of publication and mention of the grant of the patent: **28.03.2012**
(21) Application number: 05775338.6
(22) Date of filing: 20.07.2005
(51) Int. Cl.: A61N 1/372

(54) **MEDICAL DEVICE TELEMETRY ARBITRATION SYSTEM USING TIME OF RESPONSE**
REAKTIONSZEIT BEI DER VERWENDUNG EINES SYSTEMS ZUR TELEMETRISCHEN ARBITRIERUNG MEDIZINISCHER VORRICHTUNGEN
SYSTEME D'ARBITRAGE PAR TELEMETRIE POUR DISPOSITIF MEDICAL AU MOYEN DU TEMPS DE REPONSE

(30) Priority: 20.07.2004 US 589994 P
(43) Date of publication of application: 20.06.2007
(73) Proprietor: Medtronic, Inc., Minneapolis, MN 55432-5604 (US)
(72) Inventor: TORGERSON, Nathan, A., Andover, MN 55304 (US); ARNETT, Christopher, M., Champlin, MN 55316 (US); NELSON, Steven, J., Wyoming, MN 55092 (US); ALMENDINGER, Allen, D., Bloomington, MN 55438 (US)
(74) Representative: Hughes, Andrea Michelle
(86) International application number: PCT/US2005/025707
(87) International publication number: WO 2006/014713

(56) References cited:
- US-A1- 2001 031 997
- US-A1- 2002 065 509
- US-A1- 2003 220 673
- US-B1- 6 482 154

## Description

### TECHNICAL FIELD

This disclosure relates to medical device transcutaneous communication systems and methods and, more particularly, to a device arbitration system and method for transcutaneous communication with such medical devices.

### BACKGROUND

Implantable medical devices for producing a therapeutic result in a patient are well known. Examples of such implantable medical devices include implantable drug infusion pumps, implantable neurostimulators, implantable cardioverters, implantable cardiac pacemakers, implantable defibrillators and cochlear implants. Some of these devices, if not all, and other devices either provide an electrical output or otherwise contain electrical circuitry to perform their intended function.

It is common for implantable medical devices, including implantable medical devices providing an electrical therapeutic output, to utilize transcutaneous telemetry to transfer information to and from the implanted medical device. Information typically transferred to an implanted medical device includes transferring instructions or programs to the implanted medical device from an external device such as an external programmer. Information typically transferred from an implanted medical includes information regarding the status and/or performance: of the implanted medical device.

As telemetry ranges increases there is an increasing problem with communicating with multiple devices, as more than one device can be in the telemetry range of a programmer at once. If more than one implanted medical device responds to an identification command, the communication link may fail as uplink responses collide with one another.
US 2002/0065509 relates to ambulatory medical systems that include a microprocessor controlled ambulatory medical device and a separate control device that communicate via telemetry where the medical device has enhanced functionality, safety features, failure detection and/or alarming capabilities.

### SUMMARY

In order to efficiently select the desired one of a plurality of implanted medical devices, many or all of which may respond to an identification command issued by an external device, special techniques may be used.

In one aspect, the present invention provides a medical system as defined in claims 1 and 3.

In another aspect, the present invention provides a method as defined in claims 6 and 12.

In a preferred embodiment, the implanted medical device selected is primarily based upon an order in time of which the plurality of implanted medical devices respond with the uplink response.

In a preferred embodiment, the implanted medical device selected is primarily based upon which of the plurality of implanted medical devices are first to respond with the uplink response.

In a preferred embodiment, the implanted device selected is based, at least in part, upon a signal strength of the uplink response of at least one of the plurality of implanted medical devices.

In a preferred embodiment, selection of the implanted medical device favors the uplink response having a stronger of the signal strength.

In a preferred embodiment, if at least two of the plurality of medical devices respond with the uplink signal in one of the plurality uplink time slots, known ones of the plurality of medical devices cease responding to the identification command; and the identification command is resent and sending of the identification command is repeated.

In a preferred embodiment, each of the plurality of medical devices are identified based, at least in part, on the uplink signal received; if at least two of the plurality of medical devices respond with the uplink signal in one of the plurality uplink time slots, known ones of the plurality of medical devices cease responding to the identification command; and the identification command is resent and to the uplink signal is again received.

In a preferred embodiment, identification commands are continued to be resent until no two of the plurality of medical devices respond with the uplink signal in one of the plurality of uplink time slots.

In a preferred embodiment, known ones of the plurality of medical devices cease responding, for a predetermined period of time, to the identification command as a result of being instructed to cease responding.

In a preferred embodiment, known ones of the plurality of medical devices cease responding, until instructed otherwise, to the identification command.

In a preferred embodiment, at least one of the plurality of implantable medical devices determines, at least in part, in which of the plurality of time slots in which to provide the uplink response based, at least in part, upon a strength of the identification command received by the at least one of the plurality of implantable medical device.

In a preferred embodiment, the plurality of medical devices provide the uplink response in an earlier set of the plurality of time slots when the strength of identification command is above a predetermined threshold and in a later set of the plurality of time slots when the strength of identification command is below the predetermined threshold.

In a preferred embodiment, a user is instructed to move at least a portion of the external device closer to one of the plurality of implanted medical devices with which the user wishes to establish transcutaneous communication.

### BRIEF DESCRIPTION OF THE DRAWINGS

**Figure 1** shows a general environmental view 10 for an implantable neurostimulation system (INS) embodiment of the present invention.

**Figure 2** illustrates an implantable medical device implanted in a patient with an external device for transcutaneous communication with such implanted medical device.

**Figure 3** shows an external device for transcutaneous communication with an implanted medical device with two implanted medical devices within range of the external device.

**Figure 4** is a timing diagram illustrating timing of issuance of an identification command and response of acknowledgements in a plurality of time slots.

**Figure 5** is an exemplary representation of a presentation of alternative medical devices to be presented to a user.

**Figure 6** is a timing diagram illustrating timing of issuance of an identification command and response of acknowledgements in a plurality of time slots with issuance of a further silence command and the re-issuance of another identification command.

### DETAILED DESCRIPTION

The following exemplary embodiments are described, for the most part, in the context of a implantable neurostimulator having a rechargeable power source, although use of the various device arbitration system with other implantable medical devices, such as pacemakers, implantable cardiac defibrillators, defibrillators, therapeutic agent infusion devices (e.g., drug pumps), is contemplated.

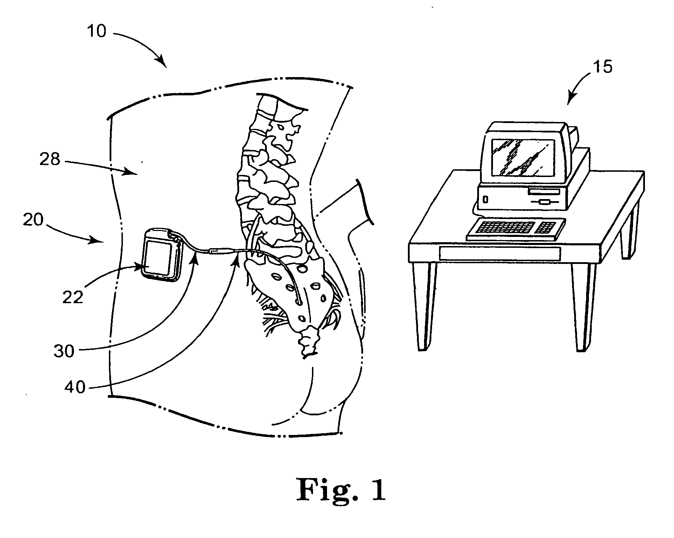

**Figure 1** shows a general, environmental view 10 for an implantable neurostimulation system (INS) embodiment. Neurostimulation systems are used to treat conditions such as pain, movement disorders, pelvic floor disorders, gastroparesis, and a wide variety of other medical conditions. Neurostimulation system 20 includes a neurostimulator 22, a stimulation lead extension 30 and a stimulation lead 40. Neurostimulator 22 is typically implanted subcutaneously in the patient's body 28 at a location selected by a clinician. Stimulation lead 40 is typically fixed in place near the location selected by the clinician using a well known device such as an adjustable anchor.

External device 15 may be used to transcutaneously communicate with implantable neurostimulator 22 as discussed below.

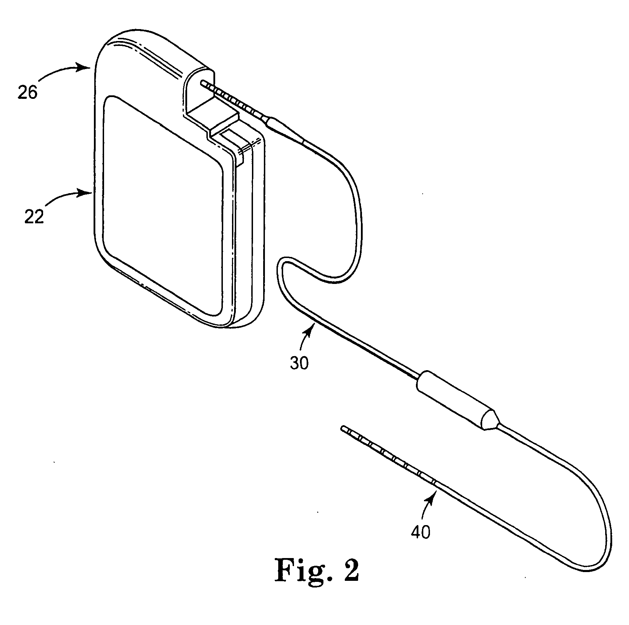

**Figure 2** shows an implantable neurostimulator 22, a stimulation lead 40, and a lead extension 30. Implantable neurostimulator 22 has a housing 24, a power supply carried in housing 24, and stimulation electronics coupled to the battery and coupled to connector block 26, which is also known as a terminal block. Stimulation lead 40 has a lead proximal end, a lead distal end and a lead body. The lead proximal end has at least one electrical connector (also known as electrical terminals) and the lead distal end has at least one stimulation electrode. There is at least one lead conductor contained in the lead body that is electrically connecting the electrical connector to the stimulation lead 40.

Neurostimulation system 20 includes an external communication device such as a physician programmer usually operable by a medical practitioner, such as a physician, and/or a patient programmer usually operable by the patient. Neurostimulation system 20 may also include or be used with various other medical devices and computer-based platforms, such as PCs, notebooks, servers, etc.

A physician programmer is typically used by a physician, clinician or other medical professional to control or set all available parameters of the implantable medical device and to set parameters and limits under which a patient may be able to control or adjust the implanted medical device.

A patient programmer is typically used by a patient, or another person caring for such patient, into whom the implantable medical device has been implanted for control of medical device parameters over which the patient typically has control. As an example, the patient could use the control to turn the implantable medical on or off or to adjust the therapy level or therapy type provided by the implanted medical device within constraints previously set by the physician programmer.

It is to be recognized and understood that the present invention may be found useful with other types of external devices, other than specifically physician programmers or patient programmers, in transcutaneous communication with implanted medical devices.

An external device typically uses well known wireless communication techniques to transcutaneously communicate with an implanted medical device. Such transcutaneous communication is typically referred to as telemetry. Many well known telemetry techniques are available to provide such transcutaneous communication.

A problem may arise, however, when an external device, such as a physician programmer or a patient programmer, attempts to communicate with an implanted medical device in a physical location which is somewhat in the proximity of a second implantable or implanted medical device. Such a situation may occur, for example, if a patient has more than one medical device implanted, if a second patient also having an implanted medical device is nearby, e.g., in an emergency ward or a hospital or other medical setting, or if another implantable medical awaiting implant, for example, is located nearby.

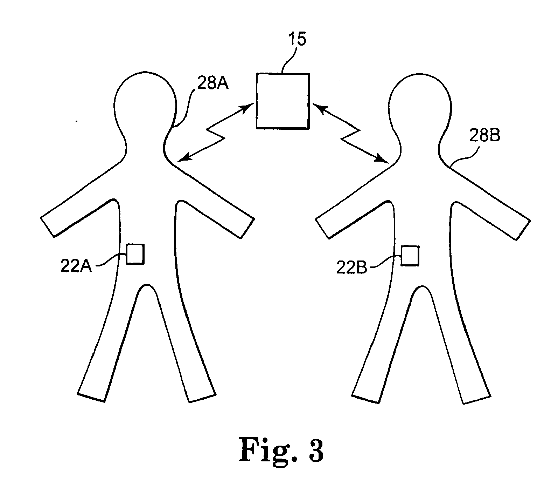

In a typical transcutaneous communication situation involving an external device and an implanted medical device, the external device will send a wireless communication request, such as an identification command. An implanted medical will receive the identification request and will send back an acknowledgement, perhaps including information about the implanted medical device. This procedure works well as long as only one implanted or implantable medical device is within wireless range of the communication request. For example, in **Figure 3** a first patient has a first neurostimulator 22A implanted in their body 28A. A second patient is located nearby, e.g., in an adjacent treatment location in a medical facility. The second patient also has an implanted medical device, in this case neurostimulator 22B, implanted in their body 28B. If both neurostimulator 22A and neurostimulator 22B operate under the same or similar telemetry protocol, both neurostimulator 22A and neurostimulator 22B may respond to the communication request with an acknowledgement. If these acknowledgements occur at the same time or near in time such as with an overlap in time, then the acknowledgement from neurostimulator 22A may collide with the acknowledgement from neurostimulator 22B and cause corruption of communication for both implanted medical devices.

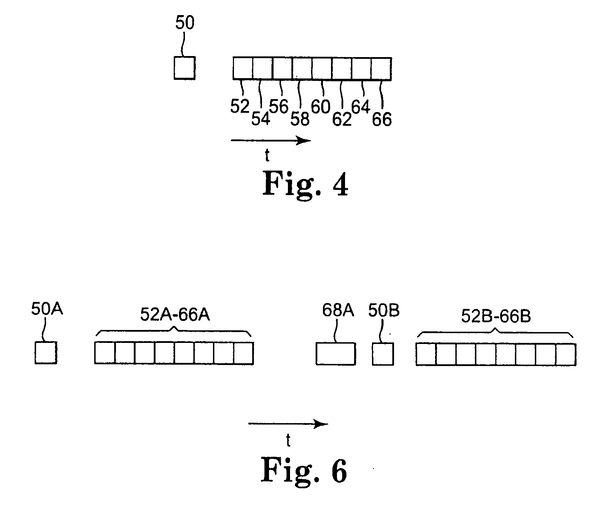

Partly for this reason, some delay is designed into the timing of the acknowledgement for an implanted medical device. A reply period may be divided into a plurality of time slots. An individual medical device, such as neurostimulator 22A or neurostimulator 22B may reply to the communication request (identification command) in one of the plurality of time slots. This is illustrated schematically in the timing diagram of **Figure 4** in which time increases from left to right in the diagram. Identification request 50 is sent by external device 15. In one of a plurality of time slots (52, 54, 56, 58, 60, 62, 64 and 66), implanted medical device 15 can respond.

If implanted medical device 15 randomly picks one of the plurality of time slots in which to respond, the chance of two or more medical devices responding in the same time slot (52, 54, 56, 58, 60, 62, 64 and 66) is reduced. If two or more medical devices do respond in the same time slot, e.g., time slot 52, then a collision occurs and communication is not established. In this case, external device 15 may reissue an identification command and repeat the process. The probability that the two or more medical devices will again randomly pick the same time slot to respond on consecutive iterations of issuance of identification commands is reduced.

However, even if communication is successful on a second or subsequent issuance of an identification command, the establishment of transcutaneous communication is delayed due to the time required to reissue the identification command and to reprocess acknowledgements. Since, in many instances, transcutaneous communication will not or can not occur during the delivery of therapy to the patient, the time periods between therapies, e.g., between electrical stimulation pulses, is in short supply. Thus, increasing the time required to establish transcutaneous communication is significant.

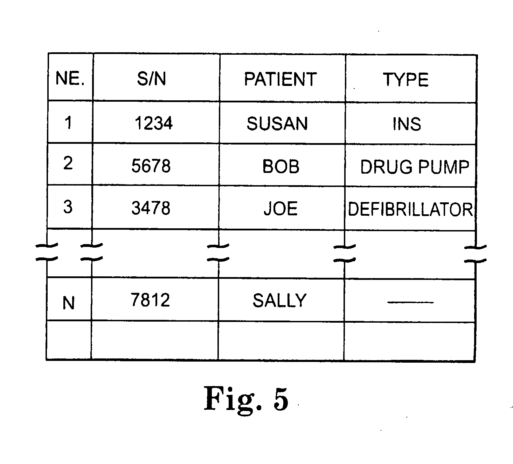

Even if two or more medical devices respond in different time slots, external device 15 must still determine which of the medical devices 22 with which to establish communication. This can be done by building a list of medical devices 22 responding to the identification request and allowing a user of the external device 15, e.g., a physician or the patient, to select the appropriate medical device 22. For example, a table, list or other presentation of information such as that illustrated in **Figure 5** could be presented to the user. As examples of information that could be presented in such a presentation, the multiple medical devices 22 identified could be listed by serial number since the serial number could have been included in the acknowledgement sent back to external device 15 by medical device 22. Similarly, the presentation could include the patient's name, again if that information was included in the acknowledgement sent back to external device 15 by medical device 22. Also, it is contemplated that the type of medical device 22 could also be presented such as whether the medical device 22 is a neurostimulator, a drug pump and a cardiac defibrillator or other medical device. It is to recognized and understood that these are merely examples of the kinds and type of information which could be presented to a user, either together, individually or selectively, enabling the user of external device 15 to make an informed decision on the selection of the medical device 15 with which to establish transcutaneous communication. It is also to be recognized and understood that other forms of presentation, other than the tabular format illustrated in Figure 5, could be used as a presentation format, such as an iconic display, visual representations by color or shape, auditory or tactile sensory presentations.

Others techniques to establish transcutaneous communication between an external device 15 and one of a plurality of medical devices 22 are contemplated.

In a first exemplary technique implanted medical devices respond to an identification command (sent by an external unit) with various random delays to prevent the uplink response from multiple implanted medical devices from overlapping and thereby corrupting the signal. The user of the external device can then look at each time slot, identify and record each implanted medical device that responds, i.e., sends an uplink response, and telemeter the identified implanted medical devices to stop communicating to an identification command from that external device for a period of time, and then reissue the identification command. After repeated attempts, all implanted medical devices within range can be found and silenced. When no further implanted medical devices respond to the identification command, a complete list of implanted medical devices within range is obtained. The user may select from the list of devices based on knowledge of how the devices are named or identified in order to uniquely select an individual implanted medical device.

This is illustrated in the timing diagram of **Figure 6** in which time increases to the right. External device 15 sends an identification command 50A. Two or more medical devices 22 respond with varying delays, each in one of time slots 52A through 66A. Any medical device 22 responding in a time slot (52A, 54A, 56A, 58A, 60A, 62A, 64A or 66A) that does not collide with the response of another medical device 22 can be identified by external device 15 and instructs with command 68A to cease responding to further identification commands, e.g., for a predetermined period of time or until another specified event or signal. External device 15 may then issue a second identification command 50B and await responses from remaining medical devices 22 in one of time slots 52B, 54B, 56B, 58B, 60B, 62B, 64B or 66B. Again any responses that do not collide identify another medical device or other medical devices 22. This process may be repeated until, eventually, no medical devices respond within any of the time slots indicating that all medical devices 22 within range have been identified. The particular medical device 22 with which external device 15 communicates may than be selected through conventional techniques, e.g., picked from a list, or other means.

A second exemplary technique uses a pseudo-random system that is based on downlink telemetry strength. Implanted medical devices that receive a strong downlink signal reply to an identification command in a first time slot, or in a first few time slots, during device arbitration. Implanted medical devices that receive a weaker downlink signal reply to the identification command in a later time slot or in later time slots. The user can then select the implanted medical device that first responds to .the identification command, which typically is the implanted medical device closest to the external device operated by the user and that received the strongest downlink signal, i.e., the strongest identification command. The user need not select the particular implanted medical device from a list of devices that are in the telemetry range of the external device. The arbitration is faster than the first exemplary technique, since it doesn't need to find and identify every implanted medical device within range, but only needs to find the closest implanted medical device, which responds in one of the earliest timeslots. Multiple command attempts are typically not needed.

In **Figure 3**, medical devices receiving an identification command having a strong or stronger downlink signal, i.e., an identification command, may respond in one of the first four time slots, namely time slots (52, 54, 56 and 58) and medical devices receiving an identification command having a weak or weaker downlink signal; i.e., an identification command, may respond in one the last four time slot, namely time slots (60, 62, 64 and 66). Relative downlink signal strength may be approximated without reference to the knowledge of the downlink signal strength of another medical device, for example, by a predetermined threshold. A signal strength of an identification command above a predetermined threshold is treated as a strong downlink signal while an identification command having a signal strength below the predetermined threshold is treated as a weak downlink signal.

This technique works well when external device 15 is located significantly closer to the medical device 22 with which communication is intended than other medical devices within communication range. An additional benefit of this technique is that if only one medical device 22 responds in a time slot indicative of a strong downlink signal, and perhaps if the user is confident that external device 15 is positioned closest to the intended medical device 22, then communication may be established with that medical device 22 without the necessity of identifying or listing all responding medical devices 22.

External device 15 will start a transcutaneous communication session with the first implanted medical device 22A recognized after broadcasting an identification command. As noted above, the uplink time slots (52, 54, 56, 58, 60, 62, 64 and 66) may be grouped according to detected downlink strength.

External device 15 may send an identification command 50 to find a medical device 22 on the first button pressed by a user that requires telemetry. The delayed uplinks from the medical device 22 may be grouped so that the medical device 22 that receives the identification command 50 with high downlink strength (for example, data strength of 7), it will send an uplink response randomized in the first four timeslots (52, .54, 56 and 58) (100-400 milliseconds of delay) and if the medical device 22 receives the identification command 50 with medium or low downlink strength (for example, data strength of 3 or 1), it will respond with a randomized uplink in the last four timeslots (60, 62, 64, and 66) (500-800 milliseconds of delay).

A medical device 22 detects a high strength downlink if an external device 22 is positioned properly over the medical device 22. Another medical device 22 that is sitting right next to the first medical device 22 will get a strength indicator of medium or low. This dramatically increases the odds of selecting the medical device 22 that is closest to the external device and can shorten the amount of time (up to 400 milliseconds) for determining which medical device 22 to talk to for a properly located external device 15.

If two implanted medical devices are at an approximately equal distance from the external device, special provision may need to be made since collision of uplink responses from the multiple implanted medical devices is likely. First, the system may still pick the first implanted medical device that communicates with it, since there still can be timeslots within each downlink strength that are still randomly sorted, or sorted by serial number, for example, so uplink responses from equally spaced implanted medical devices do not collide. Second, the user may by alerted to move the external device closer to the implanted medical device with which communication is desired, in the event of collision of uplink responses from more than one implanted medical device. Third, all of the implanted medical devices may be listed as in the first exemplary technique described above and the user may select the implanted medical device with which communication is desired.

In a third exemplary technique, the user may look at the strength of the uplink signal from each of the plurality of implanted medical devices. In this exemplary embodiment, time-slot arbitration would be used with many different time slots to minimize and, hopefully, prevent uplink response collisions. The user may receive uplink response from all of the implanted medical devices using the arbitration techniques as described above in the first exemplary technique and then look to see which implanted medical device replied with the strongest uplink signal. The user may then select the implanted device having the strongest uplink response signal, which is usually the implanted medical device that is closest to the external device issuing the identification command.

It is to be recognized and understood that the above described techniques are primarily intended to arbitrate between two or more implanted medical devices in the establishment and/or identification of such devices by an external device desiring to communicate with one or more such devices. It should be recognized that once each, or one of, the implanted medical are identified such that an identifier associated with the implanted medical device is known to the external device, that the external device may communicate with any such known implanted medical device simply by specifying the identifier or address of that device without the necessity to further resort to arbitration techniques.

After the communication session starts, the ID medical device 22 selected is set in the external device 15 and the external device 15 will only communicate to the initial medical device 22 found until the communication session is ended.

Thus, embodiments of the medical device telemetry arbitration system are disclosed. One skilled in the art will appreciate that the present invention can be practiced with embodiments other than those disclosed. The disclosed embodiments are presented for purposes of illustration and not limitation, and the present invention is limited only by the claims that follow.

## Claims

1. A medical device communication system, comprising:
a plurality of implantable medical devices (22); and
an external device (15) being configured to transcutaneously send an identification command to at least one of said plurality of implantable medical devices;
said plurality of implantable medical devices being configured to respond to said identification command with an uplink response in one of a plurality of uplink time slots;
said external device being configured to receive said uplink response from each of said at least one of said plurality of implanted medical devices;
**characterized in that** said external device is configured to establish transcutaneous communication to a selected one of said plurality of implanted medical devices based primarily upon an order in time of which said plurality of implanted medical devices respond with said uplink response.

2. A medical device communication system as in claim 1 wherein said external device is configured to select primarily based upon which of said plurality of implanted medical devices are first to respond with said uplink response.

3. A medical device communication system, comprising:
a plurality of implantable medical devices (22); and
an external device (15) being configured to transcutaneously send an identification command to at least one of said plurality of implantable medical devices;
said plurality of implantable medical devices being configured to respond to said identification command with an uplink response in one of a plurality of uplink time slots;
said external device being configured to receive said uplink response from each of said at least one of said plurality of implanted medical devices;
wherein said external device is configured to establish transcutaneous communication to a selected one of said plurality of implanted medical devices based, at least in part, upon an order in time of which said plurality of implanted medical devices respond with said uplink response;
**characterised in that** at least one of said plurality of implanted medical devices is configured to determine, at least in part, in which of said plurality of time slots in which to provide said uplink response based, at least in part, upon a strength of said identification command received by said at least one of said plurality of implantable medical device.

4. A medical device communication system as in claim 10 wherein said plurality of medical devices are configured to provide said uplink response in an earlier set of said plurality of time slots when said strength of identification command is above a predetermined threshold and in a later set of said plurality of time slots when said strength of identification command is below said predetermined threshold.

5. A medical device communication system as in claim 1 wherein said external device is further configured to instruct a user to move at least a portion of said external device closer to one of said plurality of implanted medical devices with which said user wishes to establish transcutaneous communication.

6. A method of selecting one of a plurality of implanted medical devices with which to establish transcutaneous communication from an external device associated with at least one of said plurality of medical devices, comprising the steps of:
sending an identification command from said external device;
at least one of said plurality of implanted medical devices responding to said identification command with an uplink response in one of a plurality of uplink time slots;
receiving said uplink response from each of said at least one of said plurality of implanted medical devices with said external device;
**characterised by** selecting one said plurality of implanted medical devices to establish transcutaneous communication based primarily on upon an order in time of which said plurality of implanted medical devices respond with said uplink response.

7. A method as in claim 6 wherein, if at least two of said plurality of medical devices respond with said uplink signal in one of said plurality uplink time slots, instructing known ones of said plurality of medical devices to cease responding to said identification command; and repeating said sending step, said responding step and said receiving step.

8. A method as in claim 6 further comprising the steps of
identifying each of said plurality of medical devices based, at least in part, on said uplink signal received;
if at least two of said plurality of medical devices respond with said uplink signal in one of said plurality uplink time slots, instructing known ones of said plurality of medical devices to cease responding to said identification command; and
repeating said sending step, said responding step and said receiving step.

9. A method as in claim 8 wherein said repeating step occurs until no two of said plurality of medical devices respond with said uplink signal in one of said plurality of uplink time slots.

10. A method as in claim 8 wherein said known ones of said plurality of medical devices cease responding to said identification command as a result of instructing step for a predetermined period of time.

11. A method as in claim 8 wherein said known ones of said plurality of medical devices cease responding to said identification command as a result of instructing step until instructed otherwise.

12. A method of selecting one of a plurality of implanted medical devices with which to establish transcutaneous communication from an external device associated with at least one of said plurality of medical devices, comprising the steps of:
sending an identification command from said external device;
at least one of said plurality of implanted medical devices responding to said identification command with an uplink response in one of a plurality of uplink time slots;
receiving said uplink response from each of said at least one of said plurality of implanted medical devices with said external device;
selecting one said plurality of implanted medical devices to establish transcutaneous communication based, at least in part, upon an order in time of which said plurality of implanted medical devices respond with said uplink response;
**characterised in that** at least one of said plurality of implantable medical devices determine, at least in part, in which of said plurality of time slots in which to provide said uplink response based, at least in part, upon a strength of said identification command received by said at least one of said plurality of implantable medical device.

13. A method as in claim 6 wherein said plurality of medical devices provide said uplink response in an earlier set of said plurality of time slots when said strength of identification command is above a predetermined threshold and in a later set of said plurality of time slots when said strength of identification command is below said predetermined threshold.

14. A method as in claim 6 further comprising the step of instructing a user to move at least a portion of said external device closer to a selected one of said plurality of implanted medical devices with which said user wishes to establish transcutaneous communication.

## Patentansprüche

1. Kommunikationssystem für medizinische Vorrichtungen, mit:
mehreren implantierbaren medizinischen Vorrichtungen (22); und
einer externen Vorrichtung (15), die konfiguriert ist, um einen Identifizierungsbefehl zu wenigstens einer der mehreren implantierbaren medizinischen Vorrichtungen transkutan zu senden;
wobei die mehreren implantierbaren medizinischen Vorrichtungen konfiguriert sind, um auf den Identifizierungsbefehl mit einer Uplink- bzw. Aufwärtsstreckenantwort in einem von mehreren Aufwärtsstreckenzeitschlitzen zu antworten;
wobei die externe Vorrichtung konfiguriert ist, um die Aufwärtsstreckenantwort von jeder der wenigstens einen der mehreren implantierten medizinischen Vorrichtungen zu empfangen;
**dadurch gekennzeichnet, dass** die externe Vorrichtung konfiguriert ist, um eine transkutane Kommunikation mit einer Ausgewählten der mehreren implantierten medizinischen Vorrichtungen hauptsächlich auf der Grundlage einer zeitlichen Reihenfolge, in der die mehreren implantierten medizinischen Vorrichtungen mit der Aufwärtsstreckenantwort antworten, aufzubauen.

2. Kommunikationssystem für medizinische Vorrichtungen nach Anspruch 1, wobei die externe Vorrichtung konfiguriert ist, um hauptsächlich auf der Grundlage der Tatsache, welche der mehreren implantierten medizinischen Vorrichtungen als erstes mit der Aufwärtsstreckenantwort antworten, auszuwählen.

3. Kommunikationssystem für medizinische Vorrichtungen, mit:
mehreren implantierbaren medizinischen Vorrichtungen (22); und
einer externen Vorrichtung (15), die konfiguriert ist, um einen Identifizierungsbefehl zu wenigstens einer der mehreren implantierbaren medizinischen Vorrichtungen transkutan zu senden;
wobei die mehreren implantierbaren medizinischen Vorrichtungen konfiguriert sind, um auf dem Identifizierungsbefehl mit einer Aufwärtsstreckenantwort in einem von mehreren Aufwärtsstreckenzeitschlitzen zu antworten;
wobei die externe Vorrichtung konfiguriert ist, um die Aufwärtsstreckenantwort von jeder der wenigstens einen der mehreren implantierten medizinischen Vorrichtungen zu empfangen;
wobei die externe Vorrichtung konfiguriert ist, um eine transkutane Kommunikation mit einer Ausgewählten der mehreren implantierten medizinischen Vorrichtungen wenigstens teilweise auf der Grundlage einer zeitlichen Reihenfolge, in der die mehreren implantierten medizinischen Vorrichtungen mit der Aufwärtsstreckenantwort antworten, aufzubauen;
**dadurch gekennzeichnet, dass** wenigstens eine der mehreren implantierten medizinischen Vorrichtungen konfiguriert ist, um wenigstens teilweise anhand einer Stärke des Identifizierungsbefehls, der von der wenigstens einen der mehreren implantierbaren medizinischen Vorrichtungen empfangen wird, wenigstens teilweise zu bestimmen, in welchen der mehreren Zeitschlitze die Aufwärtsstreckenantwort bereitgestellt werden soll.

4. Kommunikationssystem für medizinische Vorrichtungen nach Anspruch 10, wobei die mehreren medizinischen Vorrichtungen konfiguriert sind, um die Aufwärtsstreckenantwort in einer früheren Menge der mehreren Zeitschlitze bereitzustellen, wenn die Stärke des Identifizierungsbefehls über einem vorgegebenen Schwellenwert liegt, und in einer späteren Menge der mehreren Zeitschlitze bereitzustellen, wenn die Stärke des Identifizierungsbefehls unter dem vorgegebenen Schwellenwert liegt.

5. Kommunikationssystem für medizinische Vorrichtungen nach Anspruch 1, wobei die externe Vorrichtung ferner konfiguriert ist, um einen Anwender anzuweisen, wenigstens einen Teil der externen Vorrichtung näher zu der einen der mehreren implantierten medizinischen Vorrichtungen, mit denen der Anwender eine transkutane Kommunikation herstellen möchte, zu bewegen.

6. Verfahren zum Auswählen einer von mehreren implantierten medizinischen Vorrichtungen, mit denen eine transkutane Kommunikation von einer externen Vorrichtung, die wenigstens einer der mehreren medizinischen Vorrichtungen zugeordnet ist, aufgebaut werden soll, das die folgenden Schritte umfasst:
Senden eines Identifizierungsbefehls von der externen Vorrichtung;
wenigstens eine der mehreren implantierten medizinischen Vorrichtungen antwortet auf den Identifizierungsbefehl mit einer Aufwärtsstreckenantwort in einem von mehreren Aufwärtsstreckenzeitschlitzen;
Empfangen der Aufwärtsstreckenantwort von jeder der wenigstens einen der mehreren implantierten medizinischen Vorrichtungen mit der externen Vorrichtung;
**gekennzeichnet durch** Auswählen einer der mehreren implantierten medizinischen Vorrichtungen, um hauptsächlich auf der Grundlage einer zeitlichen Reihenfolge, in der die mehreren implantierten medizinischen Vorrichtungen mit der Aufwärtsstreckenantwort antworten, eine transkutane Kommunikation aufzubauen.

7. Verfahren nach Anspruch 6, wobei dann, wenn wenigstens zwei der mehreren medizinischen Vorrichtungen mit dem Aufwärtsstreckensignal in einem der mehreren Aufwärtsstreckenzeitschlitze antworten, Bekannte der mehreren medizinischen Vorrichtungen angewiesen werden, das Antworten auf den Identifizierungsbefehl zu beenden; und Wiederholen des Sendeschritts, des Antwortschritts und des Empfangsschritts.

8. Verfahren nach Anspruch 6, das ferner die folgenden Schritte umfasst:
Identifizieren jeder der mehreren medizinischen Vorrichtungen wenigstens teilweise anhand des empfangenen Aufwärtsstreckensignals;
falls wenigstens zwei der mehreren medizinischen Vorrichtungen mit dem Aufwärtsstreckensignal in einem der mehreren Aufwärtsstreckenzeitschlitze antworten, Anweisen Bekannter der mehreren medizinischen Vorrichtungen, das Antworten auf den Identifizierungsbefehl zu beenden; und
Wiederholen des Sendeschritts, des Antwortschritts und des Empfangsschritts.

9. Verfahren nach Anspruch 8, wobei der Wiederholungsschritt erfolgt, bis keine zwei der mehreren medizinischen Vorrichtungen mit dem Aufwärtsstreckensignal in einem der mehreren Aufwärtsstreckenzeitschlitze antworten.

10. Verfahren nach Anspruch 8, wobei die Bekannten der mehreren medizinischen Vorrichtungen das Antworten auf den Identifizierungsbefehl als Ergebnis des Anweisungsschritts für eine vorgegebene Zeitdauer beenden.

11. Verfahren nach Anspruch 87, wobei die Bekannten der mehreren medizinischen Vorrichtungen das Antworten auf den Identifizierungsbefehl als Ergebnis des Anweisungsschritts beenden, bis andere Anweisungen erfolgen.

12. Verfahren zum Auswählen einer von mehreren implantierten medizinischen Vorrichtungen, mit denen von einer externen Vorrichtung, die wenigstens einer der mehreren medizinischen Vorrichtungen zugeordnet ist, eine transkutane Kommunikation aufgebaut werden soll, das die folgenden Schritte umfasst:
Senden eines Identifizierungsbefehls von der externen Vorrichtung;
wenigstens eine der mehreren implantierten medizinischen Vorrichtungen antwortet auf den Identifizierungsbefehl mit einer Aufwärtsstreckenantwort in einem von mehreren Aufwärtsstreckenzeitschlitzen;
Empfangen der Aufwärtsstreckenantwort von jeder der wenigstens einen der mehreren implantierten medizinischen Vorrichtungen mit der externen Vorrichtung;
Auswählen einer der mehreren implantierten medizinischen Vorrichtungen, um eine transkutane Kommunikation wenigstens teilweise auf der Grundlage einer zeitlichen Reihenfolge, in der die mehreren implantierten medizinischen Vorrichtungen mit der Aufwärtsstreckenantwort antworten, aufzubauen;
**dadurch gekennzeichnet, dass** wenigstens eine der mehreren implantierbaren medizinischen Vorrichtungen wenigstens teilweise anhand einer Stärke des Identifizierungsbefehls, der von der wenigstens einen der mehreren implantierbaren medizinischen Vorrichtungen empfangen wird, wenigstens teilweise bestimmen, in welchem der mehreren Zeitschlitze die Aufwärtsstreckenantwort bereitgestellt werden soll.

13. Verfahren nach Anspruch 6, wobei die mehreren medizinischen Vorrichtungen die Aufwärtsstreckenantwort in einer früheren Menge der mehreren Zeitschlitze bereitstellen, wenn die Stärke eines Identifizierungsbefehls über einem vorgegebenen Schwellenwert liegt, und in einer späteren Menge der mehreren Zeitschlitze bereitstellen, wenn die Stärke des Identifizierungsbefehls unter dem vorgegebenen Schwellenwert liegt.

14. Verfahren nach Anspruch 6, das ferner den Schritt des Anweisens eines Anwenders, wenigstens einen Teil der externen Vorrichtung näher zu einer Ausgewählten der mehreren implantierten medizinischen Vorrichtungen, mit denen der Anwender eine transkutane Kommunikation aufbauen möchte, zu bewegen, umfasst.

## Revendications

1. Système de communication de dispositif médical, comportant :
une pluralité de dispositifs médicaux implantables (22) ; et
un dispositif externe (15) étant configuré pour envoyer de manière transcutanée une instruction d'identification à au moins un dispositif parmi ladite pluralité de dispositifs médicaux implantables ;
ladite pluralité de dispositifs médicaux implantables étant configurés pour répondre à ladite instruction d'identification à l'aide d'une réponse en liaison montante dans un intervalle de temps parmi une pluralité d'intervalles de temps en liaison montante ;
ledit dispositif externe étant configuré pour recevoir ladite réponse en liaison montante depuis chaque dispositif parmi ledit au moins un dispositif de ladite pluralité de dispositifs médicaux implantés ;
**caractérisé en ce que** ledit dispositif externe est configuré pour établir une communication transcutanée avec un dispositif médical sélectionné parmi ladite pluralité de dispositifs médicaux implantés basée principalement sur une chronologie temporelle selon laquelle les dispositifs médicaux implantés répondent à l'aide de ladite réponse en liaison montante.

2. Système de communication de dispositif médical selon la revendication 1, dans lequel ledit dispositif externe est configuré principalement pour effectuer une sélection sur la base de laquelle ladite pluralité de dispositifs médicaux implantés doivent tout d'abord répondre à l'aide de ladite réponse en liaison montante.

3. Système de communication de dispositif médical, comportant :
une pluralité de dispositifs médicaux implantables (22) ; et
un dispositif externe (15) étant configuré pour envoyer de manière transcutanée une instruction d'identification à au moins un dispositif parmi ladite pluralité de dispositifs médicaux implantables ;
ladite pluralité de dispositifs médicaux implantables étant configurés pour répondre à ladite instruction d'identification à l'aide d'une réponse en liaison montante dans un intervalle de temps parmi une pluralité d'intervalles de temps en liaison montante ;
ledit dispositif externe étant configuré pour recevoir ladite réponse en liaison montante en provenance de chaque dispositif parmi ledit au moins un dispositif de ladite pluralité de dispositifs médicaux implantés,
dans lequel ledit dispositif externe est configuré pour établir une communication transcutanée avec un dispositif sélectionné parmi ladite pluralité de dispositifs médicaux implantés sur la base, au moins en partie, d'une chronologie temporelle selon laquelle ladite pluralité de dispositifs médicaux implantés répondent à l'aide de ladite réponse en liaison montante ;
**caractérisé en ce que** au moins un dispositif parmi ladite pluralité des dispositifs médicaux implantés est configuré pour déterminer, au moins en partie, dans quel intervalle parmi ladite pluralité d'intervalles de temps délivrer ladite réponse en liaison montante sur la base, au moins en partie, d'une force de ladite instruction d'identification reçue par ledit au moins un dispositif de ladite pluralité de dispositifs médicaux implantables.

4. Système de communication de dispositif médical selon la revendication 10, dans lequel ladite pluralité de dispositifs médicaux sont configurés pour délivrer ladite réponse en liaison montante dans un ensemble antérieur de ladite pluralité d'intervalles de temps lorsque ladite force de l'instruction d'identification se situe au-dessus d'un seuil prédéterminé et dans un ensemble ultérieur de ladite pluralité d'intervalles de temps lorsque ladite force de l'instruction d'identification se situe au-dessous dudit seuil prédéterminé.

5. Système de communication de dispositif médical selon la revendication 1, dans lequel ledit dispositif externe est en outre configuré pour ordonner à un utilisateur de déplacer au moins une partie dudit dispositif externe de manière à ce qu'il soit plus proche d'un dispositif parmi ladite pluralité de dispositifs médicaux implantés avec lequel ledit utilisateur souhaite établir une communication transcutanée.

6. Procédé de sélection d'un dispositif parmi une pluralité de dispositifs médicaux implantés à l'aide duquel établir une communication transcutanée depuis un dispositif externe associé à au moins un dispositif parmi ladite pluralité de dispositifs médicaux, comportant les étapes consistant à :
envoyer une instruction d'identification depuis ledit dispositif externe ;
au moins l'un de ladite pluralité de dispositifs médicaux implantés répondant à ladite instruction d'identification à l'aide d'une réponse en liaison montante dans un intervalle de temps parmi une pluralité d'intervalles de temps en liaison montante ;
recevoir ladite réponse en liaison montante en provenance de chaque dispositif parmi ledit au moins un dispositif de ladite pluralité de dispositifs médicaux implantés avec ledit dispositif externe ;
**caractérisé par** la sélection d'un dispositif parmi ladite pluralité de dispositifs médicaux implantés afin d'établir une communication transcutanée basée principalement sur une chronologie temporelle selon laquelle ladite pluralité de dispositifs médicaux implantés répondent avec ladite réponse en liaison montante.

7. Procédé selon la revendication 6 dans lequel, si au moins deux dispositifs parmi ladite pluralité de dispositifs médicaux répondent à l'aide dudit signal en liaison montante dans un intervalle de temps parmi ladite pluralité d'intervalles de temps liaison montante, on ordonne aux dispositifs connus parmi ladite pluralité de dispositifs médicaux de cesser de répondre à ladite instruction d'identification ; et de répéter ladite étape d'envoi, ladite étape de réponse et ladite étape de réception.

8. Procédé selon la revendication 6 comportant en outre les étapes consistant à :
identifier chaque dispositif parmi ladite pluralité de dispositifs médicaux sur la base, au moins en partie, dudit signal en liaison montante reçu ;
si au moins deux dispositifs parmi ladite pluralité de dispositifs médicaux répondent à l'aide dudit signal en liaison montante dans un intervalle de temps parmi ladite pluralité d'intervalles de temps en liaison montante, on ordonne aux dispositifs connus parmi ladite pluralité de dispositifs médicaux de cesser de répondre à ladite instruction d'identification ; et
de répéter ladite étape d'envoi, ladite étape de réponse et ladite étape de réception.

9. Procédé selon la revendication 8, dans lequel ladite étape de répétition survient jusqu'à ce que moins de deux dispositifs parmi ladite pluralité de dispositifs médicaux répondent à l'aide dudit signal en liaison montante dans un intervalle de temps parmi ladite pluralité d'intervalles de temps en liaison montante.

10. Procédé selon la revendication 8, dans lequel lesdits dispositifs connus parmi ladite pluralité de dispositifs médicaux cessent de répondent à ladite instruction d'identification en résultat de l'étape consistant à donner un ordre pendant une période de temps prédéterminée.

11. Procédé selon la revendication 8, dans lequel lesdits dispositifs connus parmi ladite pluralité de dispositifs médicaux cessent de répondre à ladite instruction d'identification en résultat de l'étape consistant à donner un ordre jusqu'à la réception d'une nouvelle instruction.

12. Procédé de sélection d'un dispositif parmi une pluralité de dispositifs médicaux implantés avec lequel établir une communication transcutanée depuis un dispositif externe associé à au moins un dispositif parmi ladite pluralité de dispositifs médicaux, comportant les étapes consistant à :
envoyer une instruction d'identification depuis ledit dispositif externe ;
au moins un dispositif parmi ladite pluralité de dispositifs médicaux implantés répondant à ladite instruction d'identification à l'aide d'une réponse en liaison montante dans un intervalle de temps parmi une pluralité d'intervalles de temps en liaison montante ;
recevoir ladite réponse en liaison montante en provenance de chaque dispositif parmi ledit au moins un dispositif de ladite pluralité de dispositifs médicaux implantés avec ledit dispositif externe ;
sélectionner un dispositif parmi ladite pluralité de dispositifs médicaux implantés afin d'établir une communication transcutanée basée, au moins en partie, sur une chronologie temporelle selon laquelle ladite pluralité de dispositifs médicaux implantés répondent à l'aide de ladite réponse en liaison montante ;
**caractérisé en ce que** au moins un dispositif parmi ladite pluralité de dispositifs médicaux implantables détermine, au moins en partie, dans quel intervalle parmi ladite pluralité d'intervalles de temps il va délivrer ladite réponse en liaison montante sur la base, au moins en partie, d'une force de ladite instruction d'identification reçue par ledit au moins un dispositif parmi ladite pluralité de dispositifs médicaux implantables.

13. Procédé selon la revendication 6 dans lequel ladite pluralité de dispositifs médicaux délivrent ladite réponse en liaison montante dans un ensemble antérieur de ladite pluralité d'intervalles de temps lorsque ladite force de l'instruction d'identification se situe au-dessus d'un seuil prédéterminé et dans un ensemble ultérieur de ladite pluralité d'intervalles de temps lorsque ladite force de l'instruction d'identification se situe au-dessous dudit seuil prédéterminé.

14. Procédé selon la revendication 6 comportant en outre l'étape consistant à ordonner à un utilisateur de déplacer au moins une partie dudit dispositif externe de manière à ce qu'il soit plus proche d'un dispositif sélectionné parmi ladite pluralité de dispositifs médicaux implantés avec lequel ledit utilisateur souhaite établir une communication transcutanée.
